# EUROPEAN PATENT APPLICATION

(11) **EP 2 397 438 A1**
(43) Date of publication of application: **21.12.2011**
(21) Application number: 10741171.2
(22) Date of filing: 04.02.2010
(51) Int. Cl.: C01B 9/08, C01B 25/10, C01B 25/455, C07C 209/68, C07C 211/63

(54) **PROCESS FOR THE PREPARATION OF FLUORINE COMPOUND**

(30) Priority: 10.02.2009 JP 2009028753
(71) Applicant: Stella Chemifa Corporation, Osaka 541-0047 (JP)
(72) Inventor: WAKI,Masahide, Izumiotsu-shi Osaka 595-0075 (JP); SAKAI,Daisuke, Izumiotsu-shi Osaka 595-0075 (JP)
(74) Representative: Sajda, Wolf E.
(86) International application number: PCT/JP2010/051565
(87) International publication number: WO 2010/092894

(57) **Abstract**

An object of the invention is to provide a process for preparing a fluorine compound, which can easily provide the objective fluorine compound using an oxygen-containing compound as a raw material without forming water as impurities as a by-product. The process for preparing a fluorine compound according to the invention includes reacting an oxygen-containing compound, which is at least one kind selected from the group consisting of oxides, hydroxides, hydrates, carbonic acid compounds, hydrogencarbonic acid compounds, boric acid compounds, sulfuric acid compounds, sulfurous acid compounds, phosphorous acid compounds and phosphoric acid compounds of at least any one kind selected from the group consisting of metal elements, H, B, C, N, Si, P, S, As, Se, Te and halogens, at least with carbonyl fluoride to form at least a fluorine compound and carbon dioxide without forming water as a by-product.

## Description

### TECHNICAL FIELD

The present invention relates to a process for preparing a fluorine compound, which is adapted to prepare a fluorine compound by reacting an oxygen-containing compound at least with carbonyl fluoride without forming water as a by-product.

### BACKGROUND ART

There has hitherto been used, as a general process of synthesizing a fluorine compound, a process of reacting an oxide with hydrofluoric acid to give a fluorine compound (for example, Patent Document 1 shown below). However, according to this process, water may sometimes be formed as a by-product as shown in the following chemical reaction scheme (1), or a hydrate may sometimes be formed as shown in the following reaction scheme (2).

Chemical Formula 1 MₐO_{b} + nHF → aMFₓ + bH₂O (1)

MₐO_{b} + nHF → aMFₓ·bH₂O (2)

wherein M represents a metal, a non-metal element excluding oxygen, or ammonia, and a, b and x represent positive integers and have the following relationships: 1 ≦ a ≦ 6, 1 ≦ b ≦ 12, 1 ≦ x ≦ 6, and n = a · x.

Therefore, a drying step of removing water from the obtained fluorine compound is necessary. Alternatively, a roasting step of removing crystal water from a hydrate is necessary so as to give an anhydride. When these steps are carried out at a high temperature (for example, 100°C to 600 °C), there is such a disadvantage that a reverse reaction and the like of the chemical scheme arises and thus a fluorine compound is hydrolyzed.

There has also been proposed a process in which the objective fluorine compound is prepared by reacting a halogen compound such as a chlorine compound and the like with a fluorine gas (for example, Patent Document 2 shown below). However, according to the relevant process, there is a limitation on a halide which can be used as a raw material, and there is also a problem from the viewpoint of materials and costs.

Furthermore, there has been exemplified a process of synthesizing a fluorine compound by adding fluorine-containing ions to a raw material compound and reacting the mixture (for example, Patent Document 3 shown below). However, according to the relevant process, there is a limitation on the kind of usable fluorine-containing ions and there is also a large limitation on a synthesizable compound.

### Prior Art Documents

### Patent Documents

Patent Document 1: JP-A-05-004 801
Patent Document 2: JP-A-09-268 005
Patent Document 3: JP-A-2002-241 196

### DISCLOSURE OF THE INVENTION

### Problems to be Solved by the Invention

The present invention has been made in light of the above problems and an object thereof is to provide a process for preparing a fluorine compound, which can easily provide the desired objective fluorine compound using an oxygen-containing compound as a raw material without forming water as impurities as a by-product.

### Means for Solving the Problems

The inventors of the present invention have studied about a process for preparing a fluorine compound so as to solve the problems of the prior art. As a result, they have found that the above problems can be solved by employing the following constitutions, and thus completed the present invention.

That is, in order to solve the above problems, the process for preparing a fluorine compound according to the present invention includes reacting an oxygen-containing compound, which is at least one kind selected from the group consisting of oxides, hydroxides, hydrates, carbonic acid compounds, hydrogencarbonic acid compounds, boric acid compounds, sulfuric acid compounds, sulfurous acid compounds, phosphorous acid compounds and phosphoric acid compounds of at least any one kind selected from the group consisting of metal elements, H, B, C, N, Si, P, S, As, Se, Te and halogens, at least with carbonyl fluoride to form at least a fluorine compound and carbon dioxide without forming water as a by-product.

Carbonyl fluoride exhibits high reactivity to inorganic or organic oxygen-containing compounds. When such a carbonyl fluoride is reacted with an oxygen-containing compound, it is possible to form at least a fluorine compound and carbon dioxide without forming water as a by-product. As a result, drying and roasting steps for removing water are unnecessary and thus the production process can be simplified and also hydrolysis caused by drying of the fluorine compound at a high temperature can be prevented. Since the raw material is inexpensive, production costs can also be reduced.

The "oxygen-containing compound" means a compound which has an oxygen atom and atoms other than the oxygen atom, and is at least any one kind selected from the group consisting of oxides, hydroxides, hydrates, carbonic acid compounds, hydrogencarbonic acid compounds, boric acid compounds, sulfuric acid compounds, sulfurous acid compounds, phosphorous acid compounds and phosphoric acid compounds. Furthermore, the oxides and the like mean oxides containing at least any one kind selected from the group consisting of metal elements, H, B, C, N, Si, P, S, As, Se, Te and halogens.

In the above process, the fluorine compound is preferably recovered with neither drying nor roasting. It is possible to prevent the fluorine compound from hydrolyzing by performing neither drying nor roasting. As a result, it is possible to prepare a high-quality fluorine compound.

In the above process, the halogen is preferably fluorine.

Furthermore, in the above process, it is preferred that the oxygen-containing compound is Li_{α}H_{β}PO_{γ}F_{δ} (in which α, β, γ and δ represent a positive integer and satisfy the following inequality expressions:
1 ≦ α ≦ 3, 0 ≦ β ≦ 2, 1 ≦ γ ≦ 4, and 0 ≦ δ ≦ 4)
and the fluorine compound formed by the reaction with carbonyl fluoride is at least any one kind selected from the group consisting of LiPF₆, LiPO₂F₂ and LiPOF₄.

### Effects of the Invention

The present invention exerts the following effects by the means described above.
That is, according to the present invention, it is made possible to prepare a fluorine compound by reacting an oxygen-containing compound, which is at least any one kind selected from the group consisting of oxides, hydroxides, hydrates, carbonic acid compounds, hydrogencarbonic acid compounds, boric acid compounds, sulfuric acid compounds, sulfurous acid compounds, phosphorous acid compounds and phosphoric acid compounds of at least any one kind selected from the group consisting of metal elements, H, B, C, N, Si, P, S, As, Se, Te and halogens, with carbonyl fluoride to form a fluorine compound without forming water as a by-product.
As a result, drying and roasting steps for removing water are unnecessary and it is possible to prevent the fluorine compound from hydrolyzing. It is also possible to reduce production costs.

### MODE FOR CARRYING OUT THE INVENTION

The process for preparing a fluorine compound according to the present embodiment will be described in more detail in the following.
The process for preparing a fluorine compound according to the present embodiment is carried out by reacting an oxygen-containing compound with carbonyl fluoride. In other words, the oxygen-containing compound and carbonyl fluoride are introduced into a reactor and then reacted in accordance with the following chemical reaction scheme.
Thereby, it is possible to form at least the fluorine compound and carbon dioxide without forming water as a by-product. The relevant reaction scheme can be expressed as follows with the oxygen-containing compound represented, for example, by MₓO_{y}H_{z} (M represents a metal, a non-metal element excluding oxygen, or ammonia, all of x, y, and z represent a positive integer and satisfy the following inequality expressions:
1 ≦ x ≦ 3, 1 ≦ y ≦ 10, and 0 ≦ z ≦ 20).

Chemical Formula 2 aMₓO_{y}H_{z} + bCOF₂ → bCO₂ + aMₓF_{d} + cHF

wherein M represents a metal, a non-metal element excluding oxygen, or ammonia, and a, b, c, d, x, y and z each represent a positive integer and have the following relationships:
1 ≦ x ≦ 3, 1 ≦ y ≦ 10, 0 ≦ z ≦ 20, d = (2b-c/a), and c = a· z.

The oxygen-containing compound is at least any one kind selected from the group consisting of oxides, hydroxides, hydrates, carbonic acid compounds, hydrogencarbonic acid compounds, boric acid compounds, sulfuric acid compounds, sulfurous acid compounds, phosphorous acid compounds and phosphoric acid compounds of at least any one kind selected from the group consisting of metal elements, H, B, C, N, Si, P, S, As, Se, Te and halogens.

Specific examples of the oxygen-containing compound include oxides such as CaO, MgO, Al₂O₃, Na₂O, K₂O, B₂O₃, P₂O₅, SiO₂, GeO₂ As₂O₃, P₂O₃, As₂O₅, CuO and FeO; hydroxides such as Ca(OH)₂, Mg(OH)₂, Al(OH)₃, NaOH, KOH, Cu(OH)₂, Fe(OH)₂, H₃BO₃, H₃PO₄, H₃PO₃ and NH₄OH; and those which can be represented by the above formula: MₓO_{y}H_{z}, for example, oxygen-containing compounds such as H₃BO₃, H₃PO₄ and H₃PO₃.

It is also possible to use those other than the oxygen-containing compound represented by the above formula: MₓO_{y}H_{z}, Specific examples thereof include compounds containing crystal water or bound water represented by H₂O, CaCl₂·6H₂O, MgSO₄·7H₂O, AlF₃·3H₂O, LiBF₄·H₂O and the like; carbonates such as CaCO₃, MgCO₃, Al₂(CO₃)₃, Na₂CO₃, K₂CO₃, CuCO₃ and FeCO₃; and hydrogen carbonates such as Ca(HCO₃)₂, Mg(HCO₃)₂, NaHCO₃ and KHCO₃. Furthermore, POF₃, POCl₃, POBr₃, Li_{α}H_{β}PO_{γ}F_{δ} (in which α, β, γ and δ represent a positive integer and satisfy the following inequality expressions:
1 ≦ α ≦ 3, 0 ≦ β ≦ 2, 1 ≦ γ ≦ 4, and 0 ≦ δ ≦ 4),
LiBF₃(OH), NaPOF₄, NaPO₂F₂, NaBr₃(OH), KPOF₄, KPO₂F₂, KBF₃(OH), KPOCl₄, KPO₂Cl₂, KBCl₃(OH), KPOBr₄, KPO₂Br₂, KBBr₃(OH), quaternary ammonium hydroxides, quaternary phosphonium hydroxides and the like are exemplified.

Examples of Li_{α}H_{β}PO_{γ}F_{δ} include, but are not limited to, LiPO₃, LiPOF₄, and LiPO₂F₂. In the case where these oxygen compounds are used as raw materials, examples of the obtained fluorine compound include LiPF₆, LiPO₂F₂, and LiPOF₄.

Examples of the quaternary ammonium hydroxide include, but are not limited to, tetramethylammonium hydroxide, tetraethylammonium hydroxide, tetrapropylammonium hydroxide, tetrabutylammonium hydroxide, triethylmethylammonium hydroxide, tripropylmethylammonium hydroxide, and tributylmethylammonium hydroxide.

Examples of the quaternary phosphonium hydroxide include, but are not limited to, tetraalkylphosphonium hydroxides having an alkyl group of 1 to 8 carbon atoms, such as tetramethylphosphonium hydroxide, tetraethylphosphanium hydroxide, tetrapropylphosphonium hydroxide, tetrabutylphosphonium hydroxide, tetrapentylphosphonium hydroxide and tetrahexylphosphonium hydroxide; and triphenylphosphonium hydroxides such as tetraphenylphosphonium hydroxide, ethyltriphenylphosphonium hydroxide, butyltriphenylphosphonium hydroxide, pentyltriphenylphosphonium hydroxide, 2-dimethylaminoethyltriphenylphosphonium hydroxide and methoxymethyltriphenylphosphonium hydroxide.

Examples of the metal elements include, but are not particularly limited to, Ca, Mg, Al, Na, K, Cu and Fe.

In the process for preparing a fluorine compound according to the present invention, carbonyl fluoride is preferably used in an amount of 0.1-fold equivalent to 100-fold equivalent, more preferably 0.5-fold equivalent to 50-fold equivalent, and still more preferably 1-fold equivalent to 10-fold equivalent, relative to the oxygen atom in the oxygen-containing compound.
When carbonyl fluoride is used in an amount of less than 0.1-fold equivalent, carbonyl fluoride does not sufficiently react with the oxygen-containing compound and the oxygen-containing compound as a raw material remains in the product, and thus a fluorine compound may not be obtained in the objective quality or amount.
In contrast, when carbonyl fluoride is used in an amount of more than 100-fold equivalent, the size of a synthesizer increases and also loss of carbonyl fluoride increases, and thus production costs may increase.

The temperature at which the oxygen-containing compound is reacted with carbonyl fluoride is preferably from -50 °C to 500 °C, more preferably from 0 °C to 200 °C, and particularly preferably from 20 °C to 100 °C. It is not preferred that the temperature is lower than -50 °C since the rate of reaction between the oxygen-containing compound and carbonyl fluoride decreases.
Moreover, in the case where carbon dioxide and also hydrogen fluoride are formed as by-products, there arises a disadvantage that the vapor pressure of these by-product gases decreases and thus it becomes difficult to separate these gases from the objective fluorine compound. Furthermore, equipment costs increase since cold storage or a low temperature generator of a reaction vessel becomes necessary, and thus economical disadvantages arise.
In contrast, when the temperature is more than 500 °C, it is efficient since the reaction rate increases. However, equipment costs increase since heat retention or a high temperature generator of a reaction vessel becomes necessary, and thus economical disadvantages arise.

There is no particular limitation as to the pressure at which the oxygen-containing compound is reacted with carbonyl fluoride, and the pressure is preferably from 0.1 kPa to 10 MPa, and more preferably from 1 kPa to 0.5 MPa.
When the pressure is lower than 0.1 kPa, expensive equipment such as a long and large vacuum container or vacuum generator becomes necessary, and thus production costs increase.
In contrast, when the pressure is higher than 10 MPa, expensive equipment such as a long and large high-pressure reactor or a high-pressure generator becomes necessary, and thus production costs increase.

In the case where carbonyl fluoride is in the form of a gas, the relevant carbonyl fluoride may be used as it is, but may be used after appropriate dilution with an inert gas so that the content becomes 0.01 % by volume to 100 % by volume.
It is preferred to use, as the inert gas, an inert gas itself, or an inert gas containing impurities which do not undergo a reaction other than the reaction with carbonyl fluoride and/or an oxygen-containing compound to form the objective fluorine compound, and which do not pollute the fluorine compound.
Specifically, CO₂ HF, N₂, Ar, He, dry air and the like can be used alone, or two or more kinds of them can be used in combination. Impurities contained in the inert gas are not preferred since the oxygen-containing compound is reacted with carbonyl fluoride to consume carbonyl fluoride particularly when moisture is contained.
Therefore, the content of moisture in the inert gas used for dilution is preferably 100 ppm or less, more preferably 10 ppm or less, and particularly preferably 1 ppm or less.

The reaction between the oxygen-containing compound and carbonyl fluoride may be directly carried out, but the oxygen-containing compound or carbonyl fluoride, or both of them may be reacted in a state of being dissolved or dispersed in a proper solvent.
In the case where one of them is in the form of a gas and the other one is a liquid or in a liquid state where the other one is dissolved or dispersed in a solvent, the reaction can be carried out by bubbling the gas into the liquid.
There is no particular limitation on the solvent, but it is preferred to use, as the solvent, a solvent as it is, or a solvent in which impurities in the solvent do not undergo a reaction other than the reaction with carbonyl fluoride and/or an oxygen-containing compound to form the objective fluorine compound, and which do not pollute the fluorine compound. Specific examples thereof include anhydrous hydrogen fluoride.
As the impurities, an oxygen-containing compound is exemplified, moisture is particularly exemplified and the content of the relevant moisture is preferably 100 ppm or less, more preferably 10 ppm or less, and particularly preferably 1 ppm or less.

In the relevant process for preparing a fluorine compound, the preparation can be carried out by any of a batch type, continuous type and semi-batch type process. There is also no particular limitation on a reactor used in the relevant preparation process and, for example, a proper reactor such as a tank type or tower type reactor can be used.
In the case of a vapor-solid reaction in which carbonyl fluoride is used in the form of a gas and the fluorine compound is in the form of a solid, the reaction between a fluorine compound and carbonyl fluoride can be efficiently carried out using a fluidized bed system.
When the reaction is carried out in the case where the oxygen-containing compound is in the form of a liquid or dissolved in a liquid, or the oxygen-containing compound is in the form of a gas and carbonyl fluoride is in the form of a liquid dissolved in a solvent, a gas-liquid contactor such as a packed tower, a plate tower or a spray tower can be suitably used regardless of whether it is an alternating current type or a parallel current type.

In the case of using a batch type or semi-batch type process, there is no particular limitation on the time (treating time) of the reaction between an oxygen-containing compound and carbonyl fluoride. However, an optimum time capable of giving a sufficient effect of synthesis may be set according to the amount of the oxygen-containing compound to be treated, the concentration of the oxygen-containing compound, the reaction temperature, the reaction pressure, the concentration of carbonyl fluoride and the like.
Specifically, the treating time is preferably 1 minute or more and 24 hours or less. When the treating time is less than 1 minute, carbonyl fluoride does not sufficiently react with the oxygen-containing compound and thus a sufficient effect of synthesis may not be obtained. In contrast, when the treating time is more than 24 hours, the treating amount decreases and thus production costs increase.

There is no particular limitation on the process of separating carbon dioxide formed as a by-product together with the relevant fluorine compound from the obtained fluorine compound, and conventionally known various processes can be employed.

As described above, according to the process for preparing a fluorine compound of the present invention, it becomes possible to prepare a fluorine compound without forming moisture as a by-product even when an oxygen-containing compound is used as a raw material without employing an especially expensive apparatus or a complicated step. As a result, drying and roasting steps for removing water are unnecessary.
The drying step means a step of natural-drying, air-drying or heating a product so as to vaporize water contained in the product. The roasting step means, in the case where the product is a hydrate, a step of heating the product for a predetermined time so as to remove crystal water from the relevant hydrate.

### EXAMPLES

Preferred examples of the present invention will be illustratively described in detail below. Unless otherwise specified restrictively, the scope of the present invention is not intended to be limited to materials and amounts described in these examples.

### Example 1

In a stainless steel pressure vessel having an inner capacity of 1 liter equipped with a pressure gauge, 5 g of calcium hydroxide, from which adhered moisture had been removed in advance by drying, was charged, Thereafter, the vessel was evacuated to vacuum by a vacuum pump through a valve attached to the vessel. Next, 100 % by volume of a COF₂ gas was introduced into the vessel through the valve while monitoring the pressure gauge until the pressure became 0.6 MPaG, and the valve was closed.

Next, the vessel was placed in a constant temperature bath of 100 °C and heated for 2 hours. As a result, the pressure in the vessel gradually increased to reach 1.2 MPaG. Thereafter, the vessel was removed from the constant temperature bath and was left standing to cool to room temperature, and the residual gas in the vessel was released. At this time, the released gas was measured by FTIR.
As a result, although a carbonic acid gas, hydrogen fluoride and carbonyl fluoride were detected, no moisture was detected. Subsequently, the atmosphere in the vessel was completely purged with an N₂ gas and the vessel was opened, and then 5 g of a powder remaining in the vessel was collected and analyzed. As a result, the powder was identified as calcium fluoride. The purity was 98 %. No conspicuous trace of corrosion was observed in the stainless steel vessel.

### Comparative Example 1

In a stainless steel pressure vessel having an inner capacity of 1 liter equipped with a pressure gauge, 5 g of calcium hydroxide, from which adhered moisture had been removed in advance by drying, was charged. Thereafter, the vessel was evacuated to vacuum by a vacuum pump through a valve attached to the vessel. Next, 100 % by volume of a HF gas was introduced into the vessel through the valve while monitoring the pressure gauge until the pressure became 0.1 MPaG, and the valve was closed.

Next, the vessel was placed in a constant temperature bath of 100 °C and heated for 2 hours. As a result, the pressure in the vessel decreased little by little to reach 0.07 MPaG. Thereafter, the vessel was removed from the constant temperature bath and was left standing to cool to room temperature. As a result, the pressure in the vessel became a negative pressure of -0.09 MPaG. Subsequently, an N₂ gas was introduced into the vessel through the valve until the pressure became 0.2 MPaG, and then the residual gas in the vessel was released together with the N₂ gas. At this time, the released gas was measured by FTIR.
As a result, hydrogen fluoride and moisture were detected. Subsequently, the atmosphere in the vessel was completely purged with an N₂ gas and the vessel was opened, and then 7.5 g of a wet pungent powder remaining in the vessel was collected and analyzed. As a result, it was found that a main component was calcium fluoride and the purity was 70 %.

On the bottom of the stainless steel vessel, clear trace of corrosion was observed. It is estimated that moisture formed as a by-product by a reaction between calcium hydroxide and hydrogen fluoride reacted with excess hydrogen fluoride to form hydrofluoric acid, and this hydrofluoric acid caused corrosion of the inside of the stainless steel vessel.

### Example 2

In a stainless steel reaction tube having an inner diameter of 16 mm, 50 g of dried silicon dioxide was charged and heated to 100 °C. Next, a COF₂ gas diluted with an N₂ gas to a volume ratio of 50 % by volume was introduced from one end of the relevant reaction tube at a rate of 1 liter per minute. After 5 minutes, the gas discharged from the other end of the reaction tube was measured by FTIR. As a result, the relevant gas was composed of SiF₄ and CO₂ and no other component was detected.

### Comparative Example 2

In a stainless steel reaction tube having an inner diameter of 16 mm, 50 g of dried silicon dioxide was charged and heated to 100 °C. Next, a HF gas diluted with an N₂ gas to a volume ratio of 50 % by volume was introduced from one end of the relevant reaction tube at a rate of 1 liter per minute. After 5 minutes, the gas discharged from the other end of the reaction tube was measured by FTIR. As a result, SiF₄, H₂O, (SiF₃)₂O and HF were detected.

### Example 3

In a stainless steel pressure vessel having an inner capacity of 1 liter equipped with a pressure gauge, 5 g of lithium chloride monohydrate, from which adhered moisture had been removed in advance by drying, was charged. Thereafter, the vessel was evacuated to vacuum by a vacuum pump through a valve attached to the vessel. Next, 100 % of a COF₂ gas was introduced into the vessel through the valve while monitoring the pressure gauge until the pressure became 0.3 MPaG, and the valve was closed.

Next, the vessel was placed in a constant temperature bath of 100 °C and heated for 2 hours. As a result, the pressure in the vessel gradually increased to reach 0.8 MPaG. Thereafter, the vessel was removed from the constant temperature bath and was left standing to cool to room temperature, and the residual gas in the vessel was released. At this time, the released gas was measured by FTIR.
As a result, although a carbonic acid gas, hydrogen fluoride, hydrogen chloride and carbonyl fluoride were detected, moisture was not detected. Subsequently, the atmosphere in the vessel was completely purged with an
N₂ gas and the vessel was opened, and then 2 g of a powder remaining in the vessel was collected and analyzed. As a result, the powder was identified as lithium fluoride. The purity was 98 %. No conspicuous trace of corrosion was observed in the stainless steel vessel.

### Example 4

First, a stainless steel reaction tube having an inner diameter of 16 mm was maintained at 200 °C. Next, COF₂ and POF₃ diluted respectively with an N₂ gas to a volume ratio of 50 % by volume were simultaneously introduced from one end of the relevant reaction tube at a rate of 1 liter per minute. After 5 minutes, the gas discharged from the other end of the reaction tube was measured by FTIR. As a result, the relevant gas was composed of PF₅ and CO₂.

### Example 5

In a stainless steel pressure vessel having an inner capacity of 1 liter equipped with a pressure gauge, 5 g of sodium metaphosphate was charged. Thereafter, the vessel was evacuated to vacuum by a vacuum pump through a valve attached to the vessel. Next, 100 % by volume of a COF₂ gas was introduced into the vessel through the valve while monitoring the pressure gauge until the pressure became 0.05 MPaG, and the valve was closed.

Next, the vessel was placed in a constant temperature bath of 100 °C and heated for 2 hours. As a result, the pressure in the vessel gradually increased to reach 0.1 MPaG. Thereafter, the vessel was removed from the constant temperature bath and was left standing to cool to room temperature, and the residual gas in the vessel was released. At this time, the released gas was measured by FTIR.
As a result, although a carbonic acid gas and carbonyl fluoride were detected, moisture was not detected. Subsequently, the atmosphere in the vessel was completely purged with an N₂ gas and the vessel was opened, and then 6 g of a powder remaining in the vessel was collected and analyzed. As a result, the powder was identified as NaPO₂F₂. The purity was 98 %. No trace of corrosion was observed in the stainless steel vessel.

### Examples 6

In a stainless steel pressure vessel having an inner capacity of 1 liter equipped with a pressure gauge, 5 g of lithium metaphosphate was charged. Thereafter, the vessel was evacuated to vacuum by a vacuum pump through a valve attached to the vessel. Next, 100 % by volume of a COF₂ gas was introduced into the vessel through the valve while monitoring the pressure gauge until the pressure became 0.65 MPaG, and the valve was closed.

Next, the vessel was placed in a constant temperature bath of 100 °C and heated for 2 hours. As a result, the pressure in the vessel gradually increased to reach 0.9 MPaG. Thereafter, the vessel was removed from the constant temperature bath and was left standing to cool to room temperature, and the residual gas in the vessel was released. At this time, the released gas was measured by FTIR.
As a result, although a carbonic acid gas and carbonyl fluoride were detected, moisture was not detected. Subsequently, the atmosphere in the vessel was completely purged with an N₂ gas and the vessel was opened, and then 8.5 g of a powder remaining in the vessel was collected and analyzed. As a result, the powder was identified as LiPF₆. The purity was 98 %. No trace of corrosion was observed in the stainless steel vessel.

### Example 7

In a stainless steel pressure vessel having an inner capacity of 1 liter equipped with a pressure gauge, 5 g of triethylmethylammonium hydroxide was charged. Thereafter, the air in the vessel was substituted by an N₂ gas through a valve attached to the vessel. Next, 100 % by volume of a COF₂ gas was introduced into the vessel through the valve while monitoring the pressure gauge until the pressure became 0.2 MPaG, and the valve was closed.

Next, the vessel was placed in a constant temperature bath of 100 °C and heated for 2 hours. As a result, the pressure in the vessel gradually increased to reach 0.25 MPaG. Thereafter, the vessel was removed from the constant temperature bath and was left standing to cool to room temperature, and the residual gas in the vessel was released. At this time, the released gas was measured by FTIR.
As a result, although a carbonic acid gas, carbonyl fluoride and hydrogen fluoride were detected, moisture was not detected. Subsequently, the atmosphere in the vessel was completely purged with an N₂ gas and the vessel was opened, and then 5 g of a substance remaining in the vessel was collected and analyzed. As a result, the substance was identified as triethylmethylammonium fluoride. The purity was 95 %. No trace of corrosion was observed in the stainless steel vessel.

### Example 8

In a 1 liter fluororesin vessel maintained at 10 °C with an ice water bath, 500 g of anhydrous HF was charged. Next, 60 g of lithium metaphosphate, from which adhered moisture had been removed in advance by drying, was gradually added and dissolved while maintaining the vessel so that the temperature of anhydrous HF in the vessel would not exceed 10 °C.

Next, a fluororesin lid equipped with insert pipes for ventilation connected respectively through a valve and an exhaust pipe connected to a reflux condenser of-50 °C was attached to the vessel containing the anhydrous HF solution. Furthermore, the valve of the exhaust pipe was opened and 100 % by volume of a COF₂ gas was bubbled into the anhydrous HF solution from the vent pipe side.
The relevant bubbling was carried out for 40 minutes while maintaining the temperature of the anhydrous HF solution at 10 °C at a ventilation rate of 2 liters per minute. Thereafter, the valve at the vent pipe side was closed.

Subsequently, the reflux condenser was removed from the exhaust pipe and the fluororesin vessel subjected to the above treatment was put in a cooling bath of -40 °C with N₂ sealing of the end of the exhaust pipe, and then allowed to stand overnight. As a result, a crystal was precipitated. The precipitated crystal was filtered under an N₂ atmosphere so as not to be exposed to atmospheric air.
Thereby, 40 g of a crystal was obtained. This crystal was transferred to the fluororesin vessel and then dried overnight at 100 °C while passing through N₂ at a rate of 1 liter per minute so as not to be exposed to atmospheric air.

After drying, the obtained crystal was qualitatively analyzed by an X-ray diffractometer. As a result, the crystal was identified as LiPF₆, The purity was 99 % or more. The contents of free acids and moisture as impurities were 50 ppm or less and 10 ppm or less, respectively.

## Claims

1. A process for preparing a fluorine compound, comprising reacting an oxygen-containing compound, which is at least one kind selected from the group consisting of oxides, hydroxides, hydrates, carbonic acid compounds, hydrogencarbonic acid compounds, boric acid compounds, sulfuric acid compounds, sulfurous acid compounds, phosphorous acid compounds and phosphoric acid compounds of at least any one kind selected from the group consisting of metal elements, H, B, C, N, Si, P, S, As, Se, Te and halogens, at least with carbonyl fluoride to form at least a fluorine compound and carbon dioxide without forming water as a by-product.

2. The process for preparing a fluorine compound according to claim 1, wherein the fluorine compound is recovered with neither drying nor roasting.

3. The process for preparing a fluorine compound according to claim 1, wherein the halogen is fluorine.

4. The process for preparing a fluorine compound according to claim 1, wherein the oxygen-containing compound is Li_{α}H_{β}PO_{γ}F_{δ} (in which α, β, γ and δ represent a positive integer and satisfy the following inequality expressions: 1 ≦ α ≦ 3, 0 ≦ β ≦ 2,
1 ≦ γ ≦ 4, and 0 ≦ δ ≦ 4) and the fluorine compound formed by the reaction with carbonyl fluoride is at least any one kind selected from the group consisting of LiPF₆, LiPO₂F₂ and LiPOF₄.
